# EUROPEAN PATENT SPECIFICATION

(11) **EP 4 134 062 B1**
(45) Date of publication and mention of the grant of the patent: **05.11.2025**
(21) Application number: 22199578.0
(22) Date of filing: 28.02.2014
(51) Int. Cl.: A61J 1/20, A61J 1/14

(54) **SYRINGE WITH GRAVITY-ASSISTED VALVE**
SPRITZE MIT SCHWERKRAFTUNTERSTÜTZTEM VENTIL
SERINGUE AVEC SOUPAPE ASSISTÉE PAR GRAVITÉ

(30) Priority: 14.03.2013 US 201313829316
(43) Date of publication of application: 15.02.2023
(62) Divisional of application: 14711092.8
(73) Proprietor: CareFusion 303, Inc., San Diego, CA 92130 (US)
(72) Inventor: MANSOUR, George Michel, San Diego, 92130 (US); PANIAN, Tyler Devin, Naperville, 60563 (US)
(74) Representative: Epping - Hermann - Fischer

(56) References cited:
- US-A1- 2010 305 548
- US-A1- 2011 208 128

## Description

### Cross-references to Related Applications

### BACKGROUND

### Field

The present disclosure generally relates to vials and syringes, and, in particular, to vial access systems and apparatus.

### Description of the Related Art

Medications and similarly dispensed substances are typically stored in a vial that is sealed with a vial cap having an access port for injecting fluid into or removing fluid from the vial. A closure of the vial usually include a pierceable rubber stopper formed of an elastomeric material such as butyl rubber or the like. The vial cap, typically formed of metal, is crimped over the pierceable rubber stopper and a flange of the vial to hold the stopper in place in the opening of the vial. The vial cap has an opening, or access port, through which the stopper and the vial opening may be accessed. A sharp cannula, such as a needle, or the piercing end of a vial adapter is typically inserted into the access port of a vial cap to make fluid connection with the contents of a vial.

Some medications for administration, such as many types of chemotherapy preparations, are packaged and shipped in a concentrated or dehydrated form, such as, but not limited to, a concentrated liquid or a dehydrated powder. Before these dehydrated or concentrated medicaments can be administered to patients, the medicaments must be reconstituted by adding a liquid rehydration or dilution component or constituent to the concentrated or dehydrated medicament. Gases from the reconstitution process, particularly for some chemotherapy preparations, can be toxic and require a closed or non-vented arrangement during processing and administration.
US20110208128A1 discloses a drug delivery connector comprising: a housing including an open distal end, an open proximal end and a chamber in fluid communication with the open distal end and the open proximal end, the housing including a distal connection portion and a proximal connection portion for attaching the housing to a container; and a ball valve disposed within the chamber and forming a releasable seal with the open distal end to prevent fluid flow from the open proximal end to the open distal end, the ball valve movable in a proximal direction to release the releasable seal to permit fluid flow from the open proximal end to the open distal end.

### SUMMARY

The invention is defined in the claims.

It is understood that various configurations of the subject technology will become readily apparent to those skilled in the art from the disclosure, wherein various configurations of the subject technology are shown and described by way of illustration. Accordingly, the summary, drawings and detailed description are to be regarded as illustrative in nature and not as restrictive.

### BRIEF DESCRIPTION OF THE DRAWINGS

The accompanying drawings, which are included to provide further understanding and are incorporated in and constitute a part of this specification, illustrate disclosed embodiments and together with the description serve to explain the principles of the disclosed embodiments. In the drawings:
**FIG. 1A** is a longitudinal cross-sectional view illustrating an example of a vial access cap, in accordance with various aspects of the present disclosure.
**FIG. 1B** is an enlarged, longitudinal cross-sectional view illustrating an example of a gravity-assisted valve of an vial access cap, in accordance with various aspects of the present disclosure.
**FIG. 2A** is a cross-sectional view illustrating an example of a syringe, in accordance with aspects of the present disclosure.
**FIG. 2B** is an enlarged, longitudinal cross-sectional view illustrating an example of a section of a syringe, in accordance with aspects of the present disclosure.
**FIG. 2C** is an enlarged, longitudinal cross-sectional view illustrating an example of a gravity-assisted valve of a syringe, in accordance with aspects of the present disclosure.
**FIG. 3** is a cross-sectional view illustrating an example of a system for fluid communication having a vial access cap and a syringe in an upright orientation, in accordance with aspects of the present disclosure.
**FIGS. 4A-4D** are longitudinal cross-sectional views illustrating exemplary embodiments of gravity-assisted valves of a syringe and a vial access cap in an upright orientation, in accordance with aspects of the present disclosure.
**FIG. 5** is a cross-sectional view illustrating an example of a system for fluid communication having a vial access cap and a syringe in an inverted orientation, in accordance with aspects of the present disclosure.
**FIGS. 6A-6D** are longitudinal cross-sectional views illustrating exemplary embodiments of gravity-assisted valves of a syringe and a vial access cap in an inverted orientation, in accordance with aspects of the present disclosure.

### DETAILED DESCRIPTION

Whether reconstituting a medicament or removing medication from a vial for administration, it is advantageous to provide vented and non-vented arrangements that ensure no breaches of the system contaminate the medication or expel toxic gases into the ambient environment.

Certain disclosed embodiments of vial access caps, syringes, and systems for fluid communications having vial access caps and syringes incorporating one or more exemplary gravity-assisted valves help ensure that no breaches occur and proper fluid communications are maintained during the preparation of medications in various vented and non-vented arrangements and the subsequent administration of the medications.

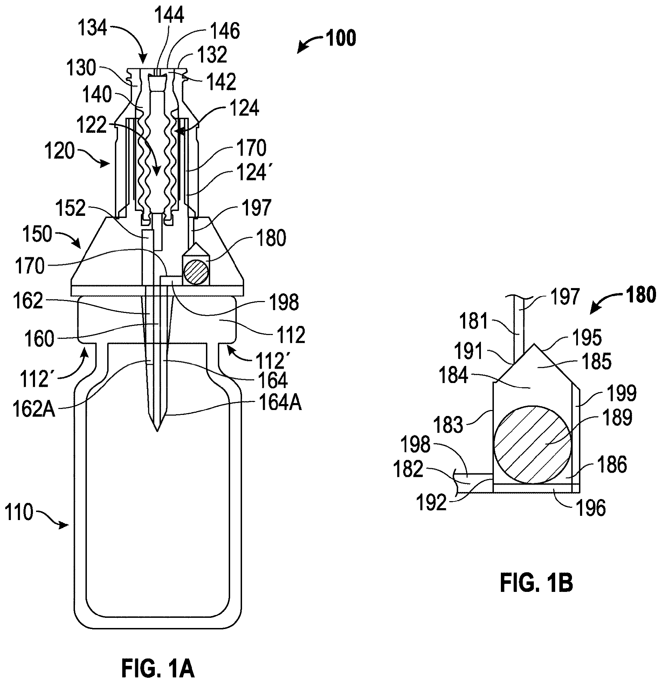

**FIG. 1A** illustrates an exemplary vial access cap 100 in accordance with certain embodiments. The vial access cap 100 is shown coupled to a vial 110 and comprises a connector 120, a base 150 including a gravity-assisted valve 180, and a canella 160. Connector 120 is a needleless connector having an opening 134 adapted to a female luer fitting in accordance with certain embodiments. However, it is understood that some embodiments of the connector 120 do not have a luer tapered fitting. The connector 120 includes a body section 130 with a top surface 132 of the opening 134.

In certain embodiments, a collapsible valve 140 is disposed within the body section 130 and has a valve tip 142 that is disposed within the opening 134 such that a valve face 146 is approximately flush with the top surface 132 of the body section 130. The valve tip 142 comprises a slit 144 that is forced closed when the valve tip 142 is positioned in a closed configuration (as illustrated in **FIG. 1A**). The slit 144 will self-open when the collapsible valve 140 is compressed and the valve face 146 is displaced, by a male luer tip for example, into a wider cavity 124 of the body section 130. The slit 144 provides access to an internal pathway 122 of the collapsible valve 140. The internal pathway 122 is operatively coupled to a fluid line 152 in the base 150. In some embodiments, cavity 124 provides an interstitial space defining a gas pathway for venting or adding a gas (e.g., sterilized air) through the base 150 and into the vial 110.

Canella 160 extends into the vial 110 and is operatively coupled to the base 150. In some embodiments, canella 160 includes a fluid lumen 162 generally used for the flow of medication or diluent and a gas lumen 164 generally used for the flow of sterilized air and/or aerosolized contents discharged during the process of reconstituting a medicament. It is to be understood that the gas lumen 164 may be used in venting and non-venting arrangements. In some embodiments, an aperture 162a of the fluid lumen 162 is disposed on the canella 160 proximal to the vial rim 112 and an aperture 164a of the gas lumen 162 is disposed on the canella 160 distal to the vial rim 112.

**FIG. 1B** provides an enlarged, longitudinal cross-sectional view of the exemplary gravity-assisted valve 180 in accordance with certain embodiments. Gravity-assisted valve 180 comprises a valve body 183 defining an elongated valve cavity 184 having generally cylindrical and conical sections. Valve 180 comprises a first port 181 proximal to a sealable end 185 having a generally smooth, ramped or conical interior wall 195 of the valve cavity 184, and a second port 192 proximal to a non-sealable end 186 having a ribbed or channelized non-sealable interior wall portion 196 (e.g., a bottom of the valve body 183 having a generally circular cross-sectional area). A valve member 189 is disposed within the elongated valve cavity 184 and movable between the sealable end 185 and non-sealable end 186. In accordance with certain embodiments, the valve member is a substantially spherical element, such as, but not limited to, a ball bearing comprising a non-reactive material with respect to the type of fluid communication for which it is to be engaged covering at least an outer surface of the ball bearing.

In certain embodiments, a ribbed or channelized longitudinal interior wall portion 199 extends along at least a portion of the generally cylindrical section of the elongated valve cavity 184. The longitudinal interior wall portion 199 generally extends from the ribbed non-sealable interior wall portion 196 to proximal the generally smooth, ramped or conical interior wall 195 of the sealable end 185. The ribs or channels along the interior longitudinal interior wall portion 199 and the interior wall portion 196 proximal the non-sealable end 186 provide a gas pathway when the valve member 189 resides in the elongated valve cavity 184 proximal to the non-sealable end 186.

The first port 181 includes a first port aperture 191 disposed on the ramped or conical interior wall 195 such that the valve member 189 will seal or block flow through the aperture 191 when the valve member 189 is in a first position proximal to the sealable end 185. The first port 181 also includes a first port conduit 197 extending to a portion of the cavity 124' of the connector 110. The second port 182 includes a second port aperture 192 disposed on the generally cylindrical section of the elongated valve cavity 184 proximal the ribbed non-sealable interior wall portion 196 such that the valve member 189 cannot seal or block flow through the aperture 192 when the valve member 189 is in a second position proximal to the non-sealable end 186.

In accordance with certain embodiments described above and illustrated in **FIGS. 1A** and **1B****,** vial access cap 100 and vial 110 are combined to provide an integrated vial design solution that drug compounders can use to encase medications for distribution to hospitals and pharmacies or the like. For example, a thin aluminum cover (not shown) or similar material encloses the vial access cap 100 (leaving access to the opening 134) and is crimped securely to the underside 112' of the vial rim 112. In this regard, the integrated vial design of the present disclosure eliminates the need for spikes, thereby reducing costs and increasing operational efficiencies for the drug administration entities. However, in some embodiments, the vial access cap 100 or various aspects thereof may be incorporated into a vial adapter for piercing the septum of a vial to obtain access to the medication therein.

In certain embodiments, one or more filters 170 are disposed at various locations along a gas pathway, for example, in the first port conduit 197 or in a portion of the cavity 124' of the connector 120. The filters 170 include a thin membrane or thickness of porous material, such as, but not limited to, polytetrafluoroethylene (PTFE) or other vinyl polymers having various pore sizes in some implementations.

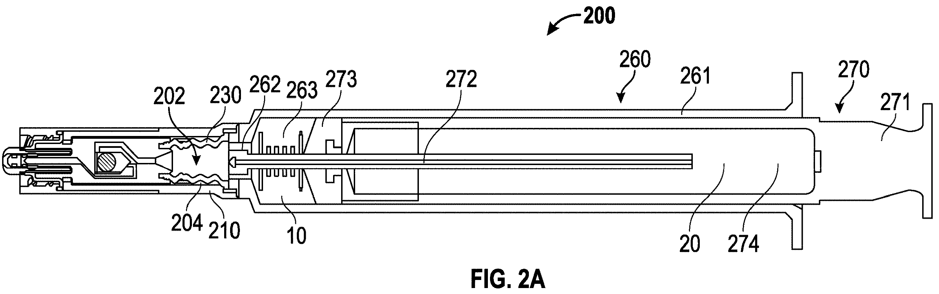

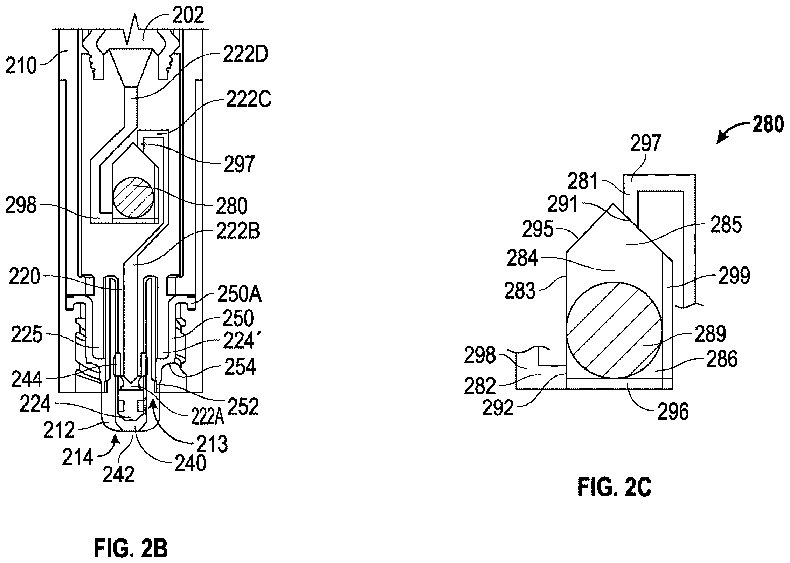

**FIGS. 2A** and **2B** illustrate an exemplary syringe 200 in accordance with certain embodiments. Syringe 200 comprises a connector body 210, a barrel 260 having a barrel tube 261, a plunger 270 having a plunger tube 271 and an interior volume 274 defining a gas reservoir, for example, a balloon (not shown) in a non-vented arrangement, or a filter (not shown) in a vented arrangement. A sealing member 273 of plunger 270, in conjunction with barrel tube 261, defines a fluid reservoir 263 and directs the flow of medication 10 (or diluent) into or out of the syringe 200. A fluid channel 262 provides a passage from the fluid reservoir 263 to the connector body 210 and a gas channel 272 provides a passage from the interior volume 274 to the connector body 210.

As shown in the enlarged, longitudinal cross-sectional view of **FIG. 2B****,** the connector body 210 of the syringe 200 has a male tip or fitting 212 with a syringe port 214. In certain embodiments, the male tip (or fitting) 212 does not have a luer taper, and therefore may provide for a gas pathway to accommodate certain venting and non-venting arrangements. A valve 220 is slidably disposed within the body 210 and partially within the male tip 212. A sealing tip 240 is disposed over a tip 224 of the valve 220. The valve 220 includes accordion bellows 230 disposed within cavity 204 distal to the valve 220. The sealing tip 240 sealingly contacts the port 214 of the male tip 212 such that the external surface 242 of the sealing tip 240 is approximately flush with the external surface of the male tip 212 around the syringe port 214. The sealing tip 240 includes a second seal 244 that forms a sliding seal between the valve 220 and the male tip 212.

A fluid pathway 222 of the connector body 210 passes through the valve 220 and the sealing tip 240. In certain embodiments, the fluid pathway 222 incorporates a gravity-assisted valve 280, and comprises a longitudinal fluid pathway portion 222d that passes from the open internal cavity 202 of the bellows 230 to a lateral second port conduit 298 of the gravity-assisted valve 280, through the gravity-assisted valve 280 to a longitudinal first port conduit 297 (having a flow direction opposite that of the longitudinal fluid pathway portion 222d). The fluid pathway 222 then extends from the longitudinal first port conduit 297 of the gravity-assisted valve 280 to a lateral fluid pathway portion 222c, from the lateral fluid pathway portion 222c to a longitudinal fluid pathway portion 222b, and from the longitudinal fluid pathway portion 222b to a lateral fluid pathway portion 122a that is open to the interior of the male tip or fitting 212. The internal cavity 202 of the connector body 210 fluidly connects to the fluid channel 262 and fluid reservoir 263.

The valve 220 includes a plurality of fingers 225 that extend toward the tip 224 of the valve 220. A sliding seal 250 is disposed over a portion of the male tip 212 and the fingers 225 with a tip 252 of the sliding seal 250 in sealing contact with a recess 213 in the male fitting 212. An end 250a of the sliding seal 250 distal to the male tip 212 is captured and secured, in accordance with some embodiments, between two components that form the connector body 210. The sliding seal 250 also has a shoulder 254 disposed proximal to the tips 224' of the fingers 124. In certain embodiments, the sliding seal 250 comprises a flexible material, such as, but not limited to, and elastomeric material.

In certain embodiments, the gravity-assisted valve 280 of the syringe 200 is similar to the valve 180 shown in **FIG. 2B****,** except that the gravity-assisted valve 280 in the syringe 200 is in the fluid path and the gravity-assisted valve 180 in the vial access cap 100 is in the gas path. In some embodiments, the gravity-assisted valve 280 of the syringe 200 is placed in other portions of the fluid pathway (e.g., fluid pathway 222) and may increase the diameter or cross-sectional area for that longitudinal portion of the syringe body (e.g., connector body 210). In other embodiments, the gravity-assisted valve 280 is an adapter or connector apparatus that is connected in series with the syringe 280 as an extension of the male tip and port assembly (e.g., syringe port 214 and male tip 212).

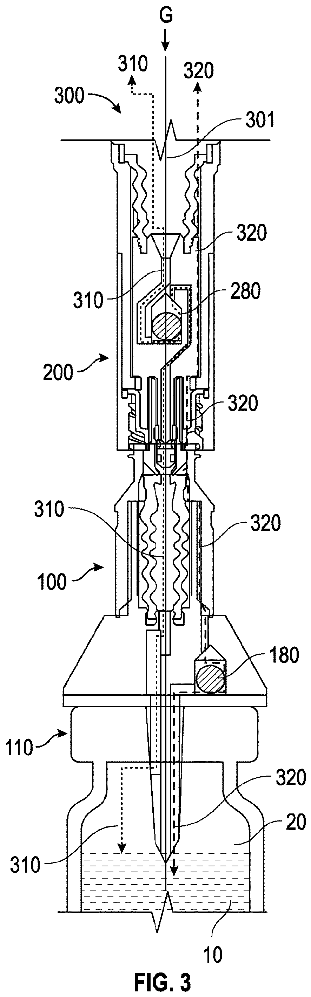

Also, it is pertinent to note the difference in conduit directions entering the first and second ports 181, 182 of the vial access gravity-assisted valve 180 and the first and second ports 281, 282 of the syringe gravity-assisted valve 280. These directional aspects with respect to the gravity-assisted valves 180, 280 have a bearing on their operation as described below with respect to **FIGS. 3****, 4A-D, 5,** and **6A-D.**

**FIG. 3** illustrates a cross-sectional view of a system 300 for fluid communication having a vial access cap 100 and a syringe 200 in an upright orientation. In certain embodiments, the vial access cap 100 is connected to a vial 110 having fluid 10 (e.g., a medication or diluent) and gas 20 therein, and the syringe 200 is operatively coupled to the vial access cap 100. A longitudinal axis 301 is defined for the system 300 showing the alignment of the gravity-assisted valves 180, 280 with respect to gravity (G). In the upright orientation, a vial's bottom surface (not shown) is proximal to the ground and the plunger 270 of the syringe 200 is distal to the ground in a generally longitudinal arrangement.

A fluid flow path 310 extends from the vial 100 through the fluid lumen 162 to the fluid reservoir 263 of the syringe 200 (e.g., through the various pathways, conduits, and channels described and illustrated in **FIGS. 1A, 1B****,** **2A****,** **2B,** and **2C****.** A gas flow path 320 extends from the vial 100 through the gas lumen 164 to the interior volume 274 of the plunger 270 of the syringe 200 (e.g., through the various pathways, conduits, and channels described and illustrated in **FIGS. 1A, 1B****,** **2A****,** **2B,** and **2C****.**

In the upright orientation, a diluent from the fluid reservoir 263 of the syringe 200 may be added to the vial 110 to reconstitute a medicament therein. Gases from the reconstitution process, particularly for some chemotherapy preparations, can be toxic and require a closed or non-vented arrangement during processing and administration of such medication. Additionally, in some non-vented arrangements, a sterilized air may be added to the vial 110 to either increase the pressure in the vial 110 to facilitate more efficient fluid flow or bring the vial 110 to a neutral or equalized pressure without contaminating the medications therein. One or more exemplary gravity-assisted valves 180, 280 incorporated into a vial access cap 100, a syringe 200, or a system having a vial access cap 100 and a syringe 200 help ensure that no breaches occur and proper fluid communications are maintained during the preparation of medications in various non-vented arrangements and vented arrangements.

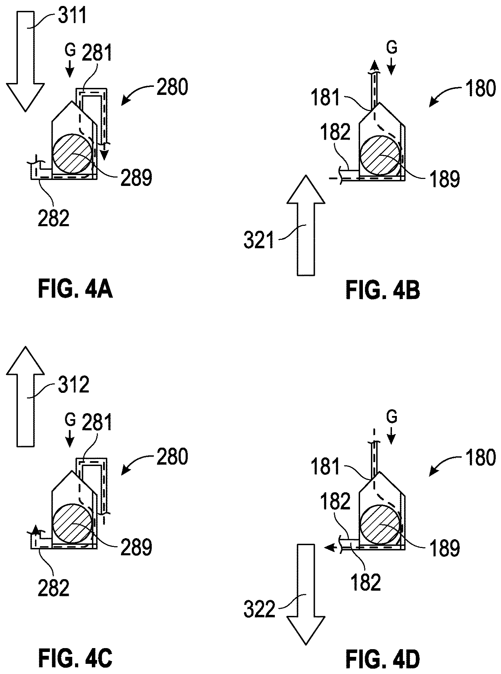

**FIGS. 4A-4D** illustrate positions of the gravity-assisted valves 180, 280 of the syringe 200 and vial access cap 100 during various operations when in the upright orientation shown in **FIG. 3****.** It is understood that, in certain embodiments, each of the valve members 189, 289 has a weight sufficient to counteract hydrostatic pressure from a gas or fluid in system 300. In general, an injection, expulsion, or suction force (or other applied or resulting force) applied to either the fluid flow path or gas flow path 320 upon the valve member 189, 289 or movement of the apparatus utilizing the gravity-assisted valve 180, 280 is required to move the valve member 189, 289 from one position to another.

In this regard, the gravity-assisted valve 180, 280 comprises at least two positions interrelated with the various operations and orientations of the system 300 in accordance with certain embodiments. For example, in a first position, the valve member 189, 289 is proximal to the sealable end 185, 285 such that the gravity-assisted valve 180, 280 is sealed at the first port 181, 281. In a second position, the valve member 189, 289 is proximal to the non-sealable end 186, 286 such that the gravity-assisted valve 180, 280 is not sealed at the first port 181, 281 and a gas or fluid can flow through the valve 180, 280.

Additionally, in the upright orientation, the gravity-assisted valves 180, 280 are biased in the second position. When biased in the second position, the gravity-assisted valves 180, 280 remain in the second position in accordance with certain aspects.

Referring to **FIG. 4A****,** when an injection force 311 is applied to the fluid flow path 310 (e.g., the plunger 270 is pushed into the barrel tube 261) while in the upright orientation, the gravity-assisted valve 280 will remain in the second position and the fluid 10 will flow from the second port 282 around the valve member 289 and out of the first port 281 in accordance with certain embodiments. Consequently, as shown in **FIG. 4B****,** when fluid 10 reaches the vial 110, an expulsion force 321 is exerted to the gas flow path 320 caused by an increase in the volume of fluid 10 (e.g., medication or diluent) entering the vial 110 or a reaction of the medicaments. As gas 20 exits the vial 110, the gravity-assisted valve 180 will remain in the second position and gas 20 will flow from the second port 182 around the valve member 189 and out of the first port 181 in accordance with certain embodiments.

**In** **FIG. 4C****,** when a suction force 312 is applied to the fluid flow path 310 (e.g., the plunger 270 is pulled away from the barrel tube 261) while in the upright orientation, the gravity-assisted valve 280 will remain in the second position and the fluid 10 will flow from the first port 281 around the valve member 289 and out of the second port 282 in accordance with certain embodiments. Consequently, as shown in **FIG. 4D****,** when fluid 10 is removed from the vial 110, a suction force 322 is applied to on the gas flow path 320 due to a reduction of volume of fluid 10 in the vial 110. As gas 20 enters the vial 110, the gravity-assisted valve 180 will remain in the second position and gas 20 will flow from the first port 181 around the valve member 189 and out of the second port 182 in accordance with certain embodiments. A similar operation of the gravity-assisted valve 180 as illustrated in **FIG. 4D** will result when a gas 20 is expelled from the interior volume 274 of the plunger 270 (e.g., sterilized air) though the gas flow path 320.

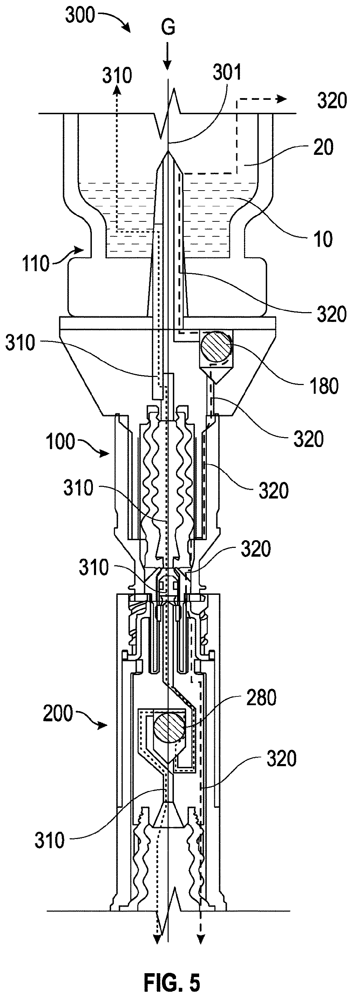

**FIG. 5** illustrates the system 300 in an inverted orientation in which the vial access cap 100 faces downwardly and is fluidly connected to the syringe 200 having its plunger 270 proximal to the ground, in accordance with certain embodiments. The inverted orientation provides an efficient manner in which medication from the vial 110 is accessed and transferred to a syringe 200, but the release of gas or fluid into the ambient environment can be hazardous and must be avoided in certain medication preparation and administration.

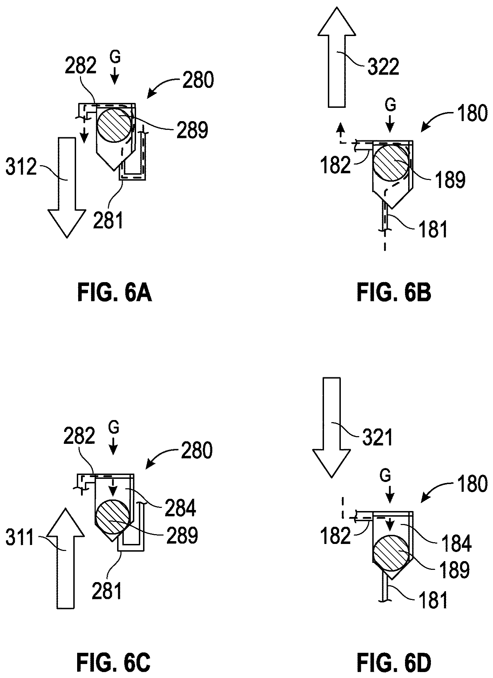

**FIGS. 6A-4D** illustrate positions of the gravity-assisted valves 180, 280 of the syringe 200 and vial access cap 100 during various operations when in the inverted orientation shown in **FIG. 5****.**

In the inverted orientation, the gravity-assisted valves 180, 280 are biased in the first position. In other words, the valve member 189, 289 is proximal to the sealable end 185, 285 such that the gravity-assisted valve 180, 280 is sealed at the first port 181, 281. When biased in the first position, the gravity-assisted valves 180, 280 may be compelled to move to the second position.

Referring now to **FIG. 6A****,** when a suction force 312 is applied to the fluid flow path 310 (e.g., the plunger 270 is pulled away from the barrel tube 261) while in the inverted orientation, the gravity-assisted valve 280 will move from the first position to the second position and the fluid 10 will flow from the first port 281 providing fluid pressure to the valve member 289 in opposition of gravity, and the fluid 10 will then flow around the valve member 289 and out of the second port 282 and into the fluid reservoir 263 in accordance with certain embodiments. In some embodiments as illustrated in **FIGS. 2C** and **6A****,** the second port aperture 292 may abut or extend into the ribbed non-sealable interior wall portion 296 so that the valve member 289 cannot fully obstruct the second port aperture 292, and the ribs or channels of the longitudinal interior wall portion 299 are arranged on a wall portion generally opposite the second port aperture 292. Hence, the resulting fluid flow extends around a longitudinally top area of the elongated valve cavity 284 and does not intersect and impede the valve member's return path to the first position into the sealable end 185 of the elongated valve cavity 284.

With reference to **FIG. 6B****,** when fluid 10 is removed from the vial 110, a suction force 322 is applied to the gas flow path 320 due to a reduction of volume of fluid 10 in the vial 110. When the suction force 322 reaches a threshold, gas 20 will be forced to enter the vial 110, thereby causing the gravity-assisted valve 180 to move from the first position to the second position. Gas 20 will flow from the first port 181 providing gas pressure to the valve member 189 in opposition of gravity, and the gas 20 will then flow around the valve member 189 and out of the second port 282 and into the vial 110 in accordance with certain embodiments.

In **FIG. 6C****,** when an injection force 311 is applied to the fluid flow path 310 (e.g., the plunger 270 is pushed into the barrel tube 261) while in the inverted orientation, the gravity-assisted valve 280 will move from the second position to the first position due to the removal of the suction force from the second port 282, the gravitational force of the valve member 289, and the injection force of the fluid 10 newly provided from the plunger 270 to the second port 282. When the gravity-assisted valve 280 is in the first position, the first port 281 will be blocked and the fluid 10 will accumulate in the elongated valve cavity and be immediately prevented from passing through fluid flow path 310 into the vial 110. Due to the proximity of the gravity-assisted valve 280 to the fluid pressure source (i.e., the injection force from the plunger 270), there is no fluid pressure build-up throughout the entire system 300 that could cause the breach of a valve, seal, conduit, or other component of the system 300 that may otherwise fail due to the pressure caused by an injection force from the plunger 270 that is not constrained.

Consequently, as shown in **FIG. 6D****,** when fluid 10 ceases to be removed from the vial 110, the suction force 322 caused upon on the gas flow path 320 likewise ceases, and the gravity-assisted valve 180 will move from the second position to the first position due to the gravitational force of valve member 189, and the expulsion force, if any, of the gas 20 or fluid 10 attempting to leave the vial 110 through the gas lumen 164. When the gravity-assisted valve 180 is in the first position, the first port 181 will be blocked and any reflux gas 20 or fluid 10 will accumulate in the elongated valve cavity 184.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

In one aspect, the term "coupled" or the like may refer to being directly coupled. In another aspect, the term "coupled" or the like may refer to being indirectly coupled.

Terms such as "top," "bottom," "front," "rear" and the like if used in this disclosure should be understood as referring to the ordinary frame of reference. Thus, a top surface, a bottom surface, a front surface, and a rear surface may extend upwardly, downwardly, diagonally, or horizontally in a frame of reference.

## Claims

1. A syringe (200) comprising:
a connector body section (210) defining a longitudinal axis and comprising,
a tip portion (212) for fluid ingress and egress; and
a gravity-assisted valve (280) comprising a valve body defining an elongated valve cavity (284) generally aligned with the longitudinal axis, a first port (281) operatively coupled to the tip portion (212), a second port (282), and a valve member (289) movable between a sealable end (285) of the elongated valve cavity (284) situated close to the first port (281) and a non-sealable end (286) of the elongated valve cavity (284) situated close to the second port (282);
a barrel section (260) generally aligned with the longitudinal axis and slidably engaged with a plunger (270), the barrel section (260) defining a fluid reservoir (263) within a volume of the barrel section (260) controllable by an end of the plunger (270) situated close to the connector body section (210), the fluid reservoir (263) operatively coupled to the second port (282) of the gravity-assisted valve (280),
wherein one of:
the gravity-assisted valve (280) is configured to allow bidirectional fluid communications between the tip portion (212) and the fluid reservoir (263) when the syringe (200) is oriented with the tip portion (212) pointing in a generally downward direction; and
the gravity-assisted valve (280) is configured to prohibit fluid communication from the fluid reservoir (263) to the tip portion (212) when the syringe (200) is oriented with the tip portion (212) pointing in a generally upward direction;
**characterized by** a gas pathway (272) extending from an interstitial space (202) of the connector body section (210) to an interior volume (274) of the plunger (270), the interior volume (274) defining a gas reservoir.

2. The syringe (200) of claim 1, wherein the sealable end of the elongated valve cavity (284) is proximal to the fluid reservoir (263) and the non-sealable end is distal to the fluid reservoir (263).

3. The syringe (200) of either of claim 1 or claim 2, wherein the tip portion (212) is non-tapered and configured for sealed engagement with a female luer fitting.

4. The syringe (200) of any of the preceding claims, wherein the valve member (289) comprises a substantially spherical element.

5. The syringe (200) of any of the preceding claims, wherein the gravity-assisted valve (280) is configured to allow fluid communication from the tip portion (212) to the fluid reservoir (263) when the syringe (200) is oriented with the tip portion (212) pointing in a generally upward direction.

6. The syringe (200) of claim 1, wherein when the gravity-assisted valve (280) is configured to allow bidirectional fluid communications between the tip portion (212) and the fluid reservoir (263) when the syringe (200) is oriented with the tip portion (212) pointing in a generally downward direction, the gravity-assisted valve (280) is also configured to prohibit fluid communication from the fluid reservoir (263) to the tip portion (212) when the syringe (200) is oriented with the tip portion (212) pointing in a generally upward direction.

7. The syringe (200) of claim 1, wherein when the gravity-assisted valve (280) is configured to prohibit fluid communication from the fluid reservoir (263) to the tip portion (212) when the syringe (200) is oriented with the tip portion (212) pointing in a generally upward direction, the gravity-assisted valve (280) is also configured to allow bidirectional fluid communications between the tip portion (212) and the fluid reservoir (263) when the syringe (200) is oriented with the tip portion (212) pointing in a generally downward direction.

## Patentansprüche

1. Spritze (200), aufweisend:
einen Verbindungskörperabschnitt (210), der eine Längsachse definiert und umfasst:
einen Spitzenabschnitt (212) für den Ein- und Austritt von Fluid; und
e in schwerkraftunterstütztes Ventil (280), das einen Ventilkörper umfasst, der einen länglichen Ventilhohlraum (284) definiert, der im Allgemeinen mit der Längsachse ausgerichtet ist, einen ersten Anschluss (281), der funktionsfähig mit dem Spitzenabschnitt (212) verbunden ist, einen zweiten Anschluss (282) und ein Ventilelement (289), das zwischen einem abdichtbaren Ende (285) des länglichen Ventilhohlraums (284), das sich in der Nähe des ersten Anschlusses (281) befindet, und einem nicht abdichtbaren Ende (286) des länglichen Ventilhohlraums (284), das sich in der Nähe des zweiten Anschlusses (282) befindet, bewegbar ist;
einem Zylinderabschnitt (260), der im Allgemeinen mit der Längsachse ausgerichtet ist und verschiebbar mit einem Kolben (270) in Eingriff steht, wobei der Zylinderabschnitt (260) einen Flüssigkeitsbehälter (263) innerhalb eines Volumens des Zylinderabschnitts (260) definiert, der durch ein Ende des Kolbens (270) steuerbar ist, das sich in der Nähe des Verbindungskörperabschnitts (210) befindet, wobei der Flüssigkeitsbehälter (263) funktionsfähig mit der zweiten Öffnung (282) des schwerkraftunterstützten Ventils (280) verbunden ist,
wobei eines von:
das schwerkraftunterstützte Ventil (280) ist so konfiguriert, dass es eine bidirektionale Fluidverbindung zwischen dem Spitzenabschnitt (212) und dem Fluidreservoir (263) ermöglicht, wenn die Spritze (200) so ausgerichtet ist, dass der Spitzenabschnitt (212) im Allgemeinen nach unten zeigt; und
das schwerkraftunterstützte Ventil (280) ist so konfiguriert, dass es eine Fluidverbindung vom Fluidreservoir (263) zum Spitzenabschnitt (212) verhindert, wenn die Spritze (200) so ausgerichtet ist, dass der Spitzenabschnitt (212) im Allgemeinen nach oben zeigt;
**gekennzeichnet durch** einen Gasweg (272), der sich von einem Zwischenraum (202) des Verbindungskörperabschnitts (210) zu einem Innenvolumen (274) des Kolbens (270) erstreckt, wobei das Innenvolumen (274) einen Gasbehälter definiert.

2. Spritze (200) nach Anspruch 1, wobei das verschließbare Ende des länglichen Ventilhohlraums (284) proximal zum Flüssigkeitsreservoir (263) und das nicht verschließbare Ende distal zum Flüssigkeitsreservoir (263) liegt.

3. Spritze (200) nach Anspruch 1 oder Anspruch 2, wobei der Spitzenabschnitt (212) nicht konisch ist und für einen dichten Eingriff mit einem weiblichen Luer-Anschluss konfiguriert ist.

4. Spritze (200) nach einem der vorstehenden Ansprüche, wobei das Ventilelement (289) ein im Wesentlichen kugelförmiges Element umfasst.

5. Spritze (200) nach einem der vorstehenden Ansprüche, wobei das schwerkraftunterstützte Ventil (280) so konfiguriert ist, dass es eine Flüssigkeitsverbindung vom Spitzenabschnitt (212) zum Flüssigkeitsreservoir (263) ermöglicht, wenn die Spritze (200) so ausgerichtet ist, dass der Spitzenabschnitt (212) im Allgemeinen nach oben zeigt.

6. Spritze (200) nach Anspruch 1, wobei, wenn das schwerkraftunterstützte Ventil (280) so konfiguriert ist, dass es eine bidirektionale Fluidverbindung zwischen dem Spitzenabschnitt (212) und dem Fluidreservoir (263) ermöglicht, wenn die Spritze (200) so ausgerichtet ist, dass der Spitzenabschnitt (212) im Allgemeinen nach unten zeigt, das schwerkraftunterstützte Ventil (280) auch so konfiguriert ist, dass es die Flüssigkeitsverbindung vom Flüssigkeitsreservoir (263) zum Spitzenabschnitt (212) verhindert, wenn die Spritze (200) so ausgerichtet ist, dass der Spitzenabschnitt (212) im Allgemeinen nach oben zeigt.

7. Spritze (200) nach Anspruch 1, wobei, wenn das schwerkraftunterstützte Ventil (280) so konfiguriert ist, dass es die Flüssigkeitsverbindung vom Flüssigkeitsreservoir (263) zum Spitzenabschnitt (212) verhindert, wenn die Spritze (200) so ausgerichtet ist, dass der Spitzenabschnitt (212) im Allgemeinen nach oben zeigt, das schwerkraftunterstützte Ventil (280) auch so konfiguriert ist, dass es eine bidirektionale Flüssigkeitsverbindung zwischen dem Spitzenabschnitt (212) und dem Flüssigkeitsreservoir (263) ermöglicht, wenn die Spritze (200) so ausgerichtet ist, dass der Spitzenabschnitt (212) im Allgemeinen nach unten zeigt.

## Revendications

1. Seringue (200) comprenant :
une section corps de connecteur (210) définissant un axe longitudinal et comprenant
une section d'embout (212) pour l'entrée et la sortie d'une fluide ; et
une valve assistée par gravité (280) comprenant un corps de valve définissant une cavité de valve allongée (284) sensiblement alignée avec l'axe longitudinal, un premier orifice (281) en liaison fonctionnelle avec la section d'embout (212), un deuxième orifice (282) et un élément de valve (289) mobile entre une extrémité étanchéifiable (285) de la cavité de valve allongée (284) située à proximité du premier orifice (281) et une extrémité non étanchéifiable (286) de la cavité de valve allongée (284) située à proximité du deuxième orifice (282) ;
une section barillet (260) sensiblement alignée avec l'axe longitudinal et engagée de manière coulissante par un plongeur (270), la section barillet (260) définissant un réservoir de fluide (263) à l'intérieur d'un volume de la section barillet (260), qui peut être commandé par une extrémité du plongeur (270) située à proximité de la section corps de connecteur (210), le réservoir de fluide (263) étant en liaison fonctionnelle avec le deuxième orifice (282) de la valve assistée par gravité (280),
l'une des affirmations suivantes étant vraie :
la valve assistée par gravité (280) est conçue pour permettre des connexions fluidiques bidirectionnelles entre la section d'embout (212) et le réservoir de fluide (263) lorsque la seringue (200) est orientée de telle sorte que la section d'embout (212) pointe dans une direction sensiblement vers le bas ; et
la valve assistée par gravité (280) est conçue pour empêcher une communication fluidique depuis le réservoir de fluide (263) vers la section d'embout (212) lorsque la seringue (200) est orientée de telle sorte que la section d'embout (212) pointe dans une direction sensiblement vers le haut ;
**caractérisée par** un chemin de gaz (272) s'étendant depuis un espace interstitiel (202) de la section corps de connecteur (210) vers un volume intérieur (274) du plongeur (270), le volume intérieur (274) définissant un réservoir de gaz.

2. La seringue (200) selon la revendication 1, sachant que l'extrémité étanchéifiable de la cavité de valve allongée (284) est située de manière proximale par rapport au réservoir de fluide (263) et l'extrémité non étanchéifiable est située de manière distale par rapport au réservoir de fluide (263).

3. La seringue (200) selon la revendication 1 ou la revendication 2, sachant que la section d'embout (212) n'est pas effilée et est conçue pour s'engager de manière étanche avec une prise femelle de type Luer.

4. La seringue (200) selon l'une quelconque des revendications précédentes, sachant que l'élément de valve (289) comprend un élément sensiblement sphérique.

5. La seringue (200) selon l'une quelconque des revendications précédentes, sachant que la valve assistée par gravité (280) est conçue pour permettre une communication fluidique depuis la section d'embout (212) vers le réservoir de fluide (263) lorsque la seringue (200) est orientée de telle sorte que la section d'embout (212) pointe dans une direction sensiblement vers le haut.

6. La seringue (200) selon la revendication 1, sachant que, lorsque la valve assistée par gravité (280) est conçue pour permettre des communications fluidiques bidirectionnelles entre la section d'embout (212) et le réservoir de fluide (263) lorsque la seringue (200) est orientée de telle sorte que la section d'embout (212) pointe dans une direction sensiblement vers le bas, la valve assistée par gravité (280) est également conçue pour empêcher une communication fluidique depuis le réservoir de fluide (263) vers la section d'embout (212) lorsque la seringue (200) est orientée de telle sorte que la section d'embout (212) pointe dans une direction sensiblement vers le haut.

7. La seringue (200) selon la revendication 1, sachant que, lorsque la valve assistée par gravité (280) est conçue pour empêcher une communication fluidique depuis le réservoir de fluide (263) vers la section d'embout (212) lorsque la seringue (200) est orientée de telle sorte que la section d'embout (212) pointe dans une direction sensiblement vers le haut, la valve assistée par gravité (280) est également conçue pour permettre des communications fluidiques bidirectionnelles entre la section d'embout (212) et le réservoir de fluide (263) lorsque la seringue (200) est orientée de telle sorte que la section d'embout (212) pointe dans une direction sensiblement vers le bas.
